(19) Europäisches Patentamt
European Patent Office
Office européen des brevets

(11) Publication number: **0 545 103 A1**

(12) **EUROPEAN PATENT APPLICATION**

(21) Application number: **92119141.7**

(22) Date of filing: **09.11.92**

(51) Int. Cl.[5]: **C07D 207/34**, C07D 207/42, C07D 403/12, C07D 413/12, C07D 417/12, C07F 9/572, A01N 43/36, A01N 43/50, A01N 43/54, A01N 43/76, A01N 43/78, A01N 43/52, A01N 43/48, A01N 47/14, A01N 47/42, A01N 47/06, A01N 47/16, A01N 57/08

(30) Priority: **04.12.91 US 804260**
**04.12.91 US 803295**

(43) Date of publication of application:
**09.06.93 Bulletin 93/23**

(84) Designated Contracting States:
**AT BE CH DE DK ES FR GB GR IE IT LI LU NL PT SE**

(71) Applicant: **AMERICAN CYANAMID COMPANY**
**1937 West Main Street P.O. Box 60**
**Stamford Connecticut 06904-0060(US)**

(72) Inventor: **Kuhn, David George**
**2 Adrian Place, Newtown**
**Pennsylvania 18940(US)**
Inventor: **Kameswaran, Venkataraman**
**34 Penn-Lyle Road, Princeton Junction**
**New Jersey 08559(US)**

(74) Representative: **Wächtershäuser, Günter, Dr.**
**Tal 29**
**W-8000 München 2 (DE)**

(54) **Insecticidal, acaricidal and molluscicidal 1-(substituted) thioalkylpyrroles.**

(57) There are provided 1-(substituted)thioalkylpyrrole compounds of formula I

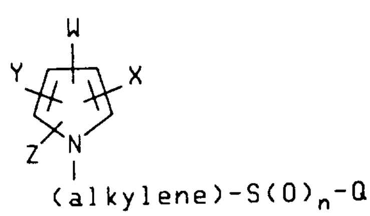

I

the use thereof for the control of fungus infestation, and insect, acarid and mollusk pests, and methods and compositions for the protection of crops from the ravages of said fungus and pests.

EP 0 545 103 A1

The present invention provides 1-(substituted)thioalkylpyrrole compounds of formula I

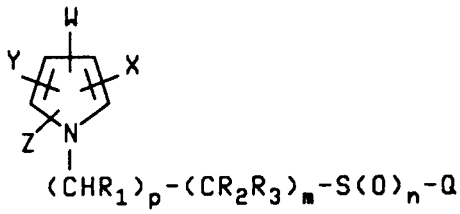

I

wherein

W is CN or $NO_2$;

X is halogen or phenyl optionally substituted with one to three $C_1$-$C_3$alkyl, $C_1$-$C_3$alkoxy, $C_1$-$C_3$alkylthio, $C_1$-$C_3$alkylsulfinyl, $C_1$-$C_3$alkylsulfonyl, halogen, CN, $NO_2$, $CF_3$, $R_4CF_2B$, $R_5CO$ or $NR_6R_7$ groups;

Y is $CF_3$, halogen or phenyl optionally substituted with one to three $C_1$-$C_3$alkyl, $C_1$-$C_3$alkoxy, $C_1$-$C_3$alkylthio, $C_1$-$C_3$alkylsulfinyl, $C_1$-$C_3$alkylsulfonyl, halogen, CN, $NO_2$, $CF_3$, $R_4CF_2B$, $R_5CO$ or $NR_6R_7$ groups;

Z is halogen or $CF_3$;

$R_1$ is hydrogen, $C_1$-$C_6$alkyl or $C_3$-$C_6$cycloalkyl;

$R_2$ and $R_3$ are each independently hydrogen, $C_1$-$C_6$alkyl or $C_3$-$C_6$cycloalkyl;

$R_4$ is hydrogen, fluorine, $CHF_2$, CHFCl or $CF_3$;

$R_5$ is $C_1$-$C_3$alkyl, $C_1$-$C_3$alkoxy or $NR_6R_7$;

$R_6$ is hydrogen or $C_1$-$C_3$alkyl;

$R_7$ is hydrogen, $C_1$-$C_3$alkyl or $R_8CO$;

$R_8$ is hydrogen or $C_1$-$c_3$alkyl;

B is $S(O)_q$ or O;

m, n, p and q are each independently an integer of 0, 1 or 2 with the proviso that the sum (p+m) must be greater than 0;

Q is 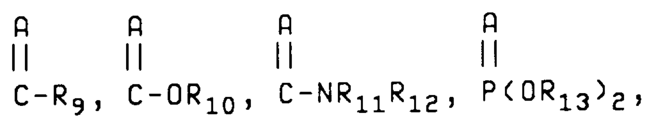

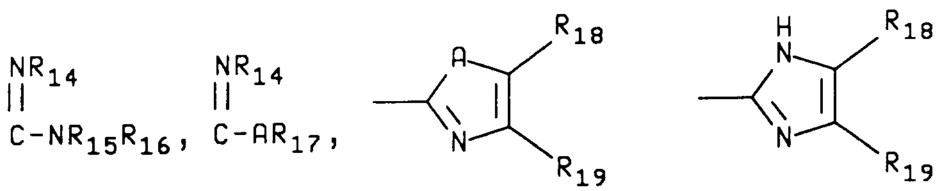

, ,

CN, $C_1$-$C_6$alkyl optionally substituted with one or more phenyl, CN or halogen groups or phenyl optionally substituted with one to three $C_1$-$C_3$alkyl, $C_1$-$C_3$alkoxy, halogen, CN, $NO_2$, $CF_3$ or $NR_{20}R_{21}$ groups;

A is O or S;

$R_9$ is $C_1$-$C_6$alkyl or phenyl;

$R_{10}$ is $C_1$-$C_6$alkyl;

$R_{11}$ and $R_{12}$ are each independently hydrogen, $C_1$-$C_6$alkyl or $-(CH_2)_n-$ wherein n is an integer of 4, 5 or 6 to form a 5- to 7- membered ring;

$R_{13}$ is $C_1$-$C_4$alkyl;

$R_{14}$ is hydrogen, $C_1$-$C_4$alkyl or $-(CH_2)_n-$ when taken together with either $R_{15}$ or $R_{17}$ wherein n is an integer

of 4, 5 or 6 to form a 5- to 7- membered ring optionally substituted with one or two $C_1$-$C_3$alkyl groups;

$R_{15}$ and $R_{16}$ are each independently hydrogen or $C_1$-$C_4$alkyl;

$R_{17}$ is $C_1$-$C_4$alkyl or $-(CH_2)_n-$ wherein n is an integer of 4, 5 or 6 when taken together with $R_{14}$ to form a 5- to 7- membered ring optionally substituted with one or two $C_1$-$C_3$alkyl groups;

$R_{18}$ and $R_{19}$ are each independently hydrogen or $C_1$-$C_3$alkyl or when taken together may form a ring wherein $R_{18}R_{19}$ is represented by $-CH=CH-CH=CH-$;

$R_{20}$ and $R_{21}$ are each independently hydrogen or $C_1$-$C_3$alkyl and

the acid addition salts thereof.

Throughout time, agriculturalists have been seeking effective and efficient means of combatting fungi, which cause harmful diseases such as mildew, blight, blast, rust, rot, in addition to insect, acarid and mollusk pests. Said fungi and pests cause considerable economic damage to essential crops when left unchecked. There is a real need in the art for alternative, effective compounds and compositions useful for agricultural applications as insecticidal, fungicidal, acaricidal and molluscicidal agents.

It is an object of the present invention to provide 1-(substituted)thioalkylpyrrole compounds that are effective agents for the control of fungus infestation and insect, acarid, and mollusk pests. It is a further object of this invention to provide methods and compositions for the protection of agronomic crops, both growing and harvested, from the ravages of said fungi and pests.

The 1-(substituted)thioalkylpyrrole compounds of the invention have the structural formula I

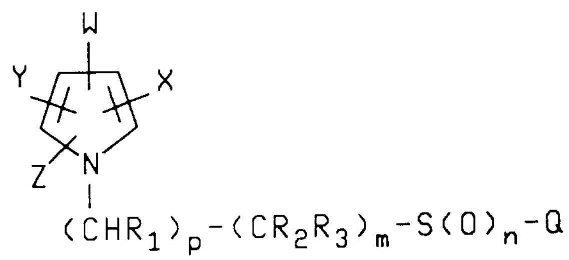

I

wherein W, X, Y, Z, $R_1$, $R_2$, $R_3$, m, n, p and Q are as described hereinabove. The term halogen, as used in the specification and claims, designates chlorine, fluorine, bromine and iodine. Acid addition salts are those known in the art such as hydrogen halides, hydrogen sulfates, sulfates, sulfonates and the like.

Preferred compounds of the invention are those having structure II

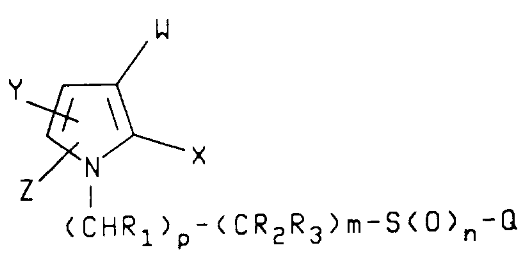

II

wherein W is CN, n is O and X, Y, Z, $R_1$, $R_2$, $R_3$, m, p and Q are as described hereinabove.

Among the formula II compounds of the present invention which are especially useful for the control of terrestial gastropods such as snails and slugs and aquatic or semi-aquatic mollusks such as cowries and limpets are those wherein W is CN, X, Y and Z are independently halogen or $CF_3$, n and m are O, p is 1, and Q is as described hereinabove.

Compounds of formula II which are particularly effective insecticidal and acaricidal agents are those wherein W is CN or $NO_2$; X is phenyl optionally substituted with one to three $C_1$-$C_3$alkyl, $C_1$-$C_3$alkoxy, $C_1$-$C_3$alkylthio, halogen or $CF_3$ groups; Y and Z are independently halogen or $CF_3$; n and m are O, p is 1, and Q is as described hereinabove.

Preferred fungicidal agents of the invention are those having structure II

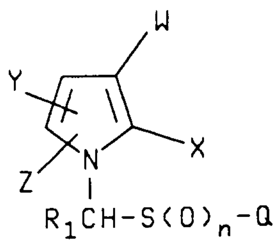

II

wherein W is CN, n is O and X, Y, Z, $R_1$ and Q are as described hereinabove.

Compounds of formula II which are particularly effective fungicidal agents are those wherein W is CN; X is halogen or phenyl optionally substituted with one to three halogen or $CF_3$ groups; Y and Z are each independently halogen; $R_1$ is hydrogen; n is O and Q is CN, phenyl optionally substituted with one to three halogen or $CF_3$ groups or

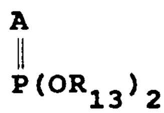

wherein A and $R_{13}$ are described hereinabove.

Certain 1-(substituted)thioalkylpyrrole compounds of formula I may be prepared by reacting a pyrrole compound of formula III with a suitable dithiocarbamate, thiocarbamate, thiophosphate or mercaptate alkali metal salt. A specific example of this reaction is illustrated in flow diagram I.

Flow Diagram I

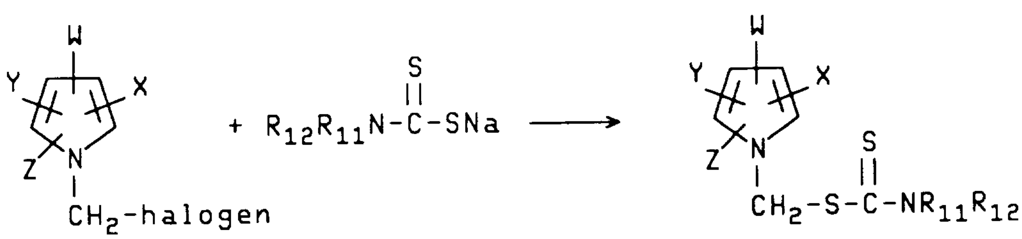

III

Moreover, certain compounds of formula I may be prepared by reacting the appropriate halomethyl thioether with a pyrrole compound of formula IV in the presence of a base. A representative example is shown in flow diagram II

Flow Diagram II

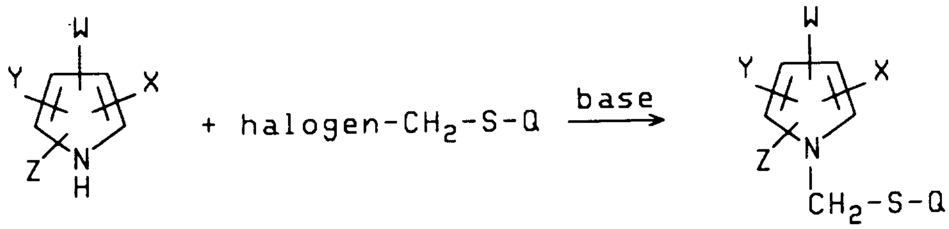


IV

Compounds of formulas III and IV are described in U.S. patent 5,010,098 and incorporated herein by reference thereto.

Other compounds of formula I may be prepared by reacting a 1-(halomethyl)pyrrole compound of formula III with a suitable thiourea reagent. In this reaction scheme, the product may be isolated as its acid addition salt. The free base may be obtained using standard procedures such as treatment with excess aqueous base and filtration or extraction of the desired product with a suitable solvent. An illustrative example is shown in flow diagram III.

Flow Diagram III

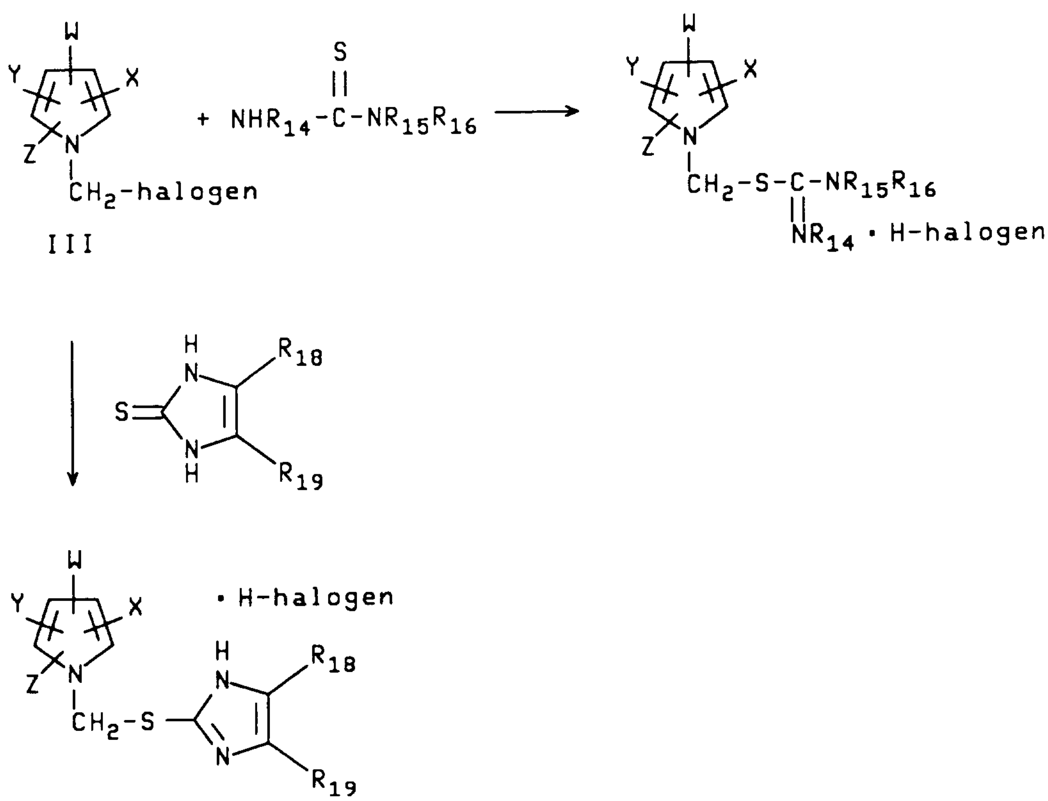

III

Further compounds of formula I may be prepared by reacting a pyrrole compound of formula IV with a halomethyl compound of formula V wherein $R_1$ is defined hereinabove in the presence of a base such as an alkali metal alkoxide to form the pyrrole intermediate of formula VI. The formula VI intermediate may then

be halogenated using conventional halogenating reagents such as N-bromosuccinimide (NBS) to give the desired 1-(haloalkyl)pyrrole second intermediate of formula VII which may then be reacted with an alkali metal thiocarbamate, dithiocarbamate, mercaptate, thiophosphate or the like to yield the 1-(substituted)-thioalkylpyrrole product as shown in flow diagram IV.

Flow Diagram IV

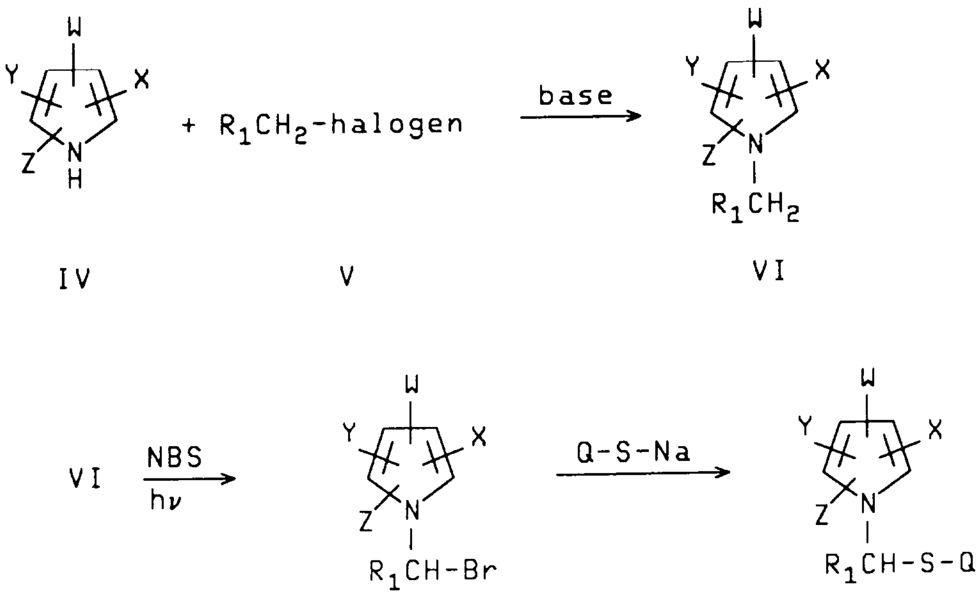


IV V VI

VII

Yet further pyrrole compounds of the invention may be prepared by reacting the formula IV pyrrole with an epoxide of formula VIII in the presence of a base to give the β-hydroxy pyrrole intermediate of formula IX which can then be converted to the corresponding tosylate of formula X by reaction with p-toluenesulfonyl chloride (tsCl) in the presence of an organic base such as pyridine. The tosylate intermediate of formula X may then be reacted with an alkali metal thiocarbamate, dithiocarbamate, mercaptate, thiophosphate or the like to yield the desired 1-(substituted)thioalkylpyrrole product as shown in flow diagram V.

Flow Diagram V

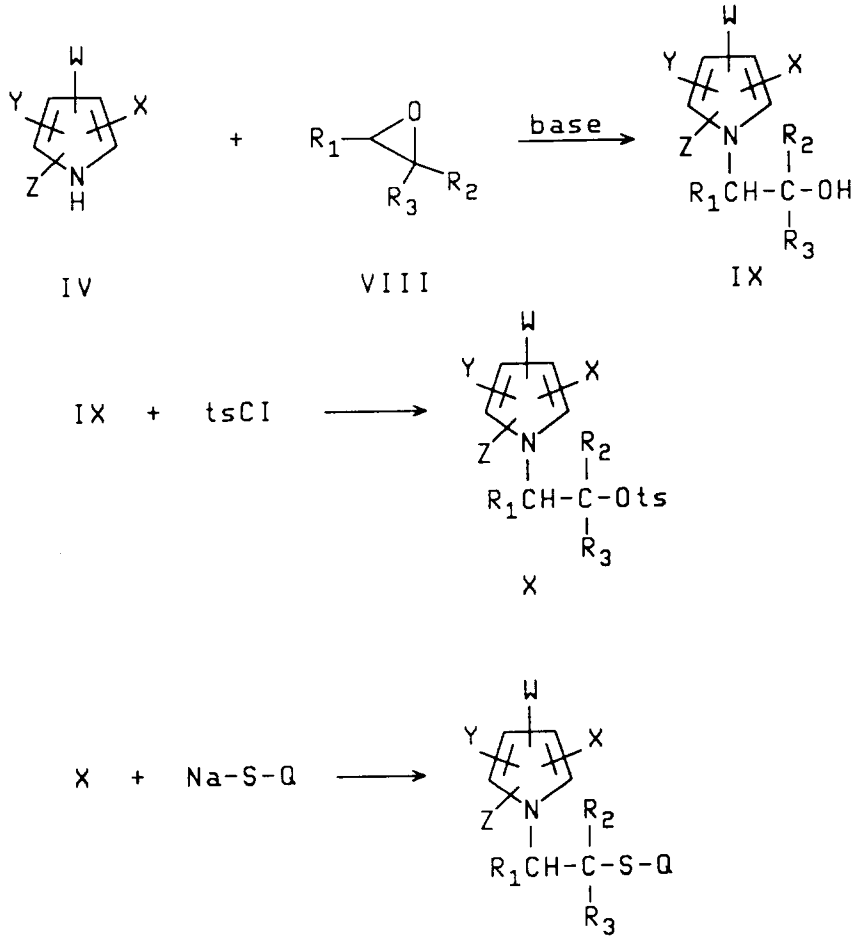


IV VIII IX X

Specific examples are provided below for the purpose of illustration. The examples utilize the above reaction schemes and also provide further means for preparing even more compounds of the invention which are not specifically described hereinabove.

The effective amount of the 1-(substituted)thioalkylpyrroles of the invention will vary with the virility of the target fungus or pest, the environment and method of treatment, and the like. In practice, generally about 10ppm to 10,000ppm, preferably about 50 ppm to 500 ppm for fungus infestation, and preferably about 100 ppm to 5,000ppm for insect, acarid or mollusk pests, of the formula I compound dispersed in a liquid carrier, when applied to the plants, seed or tuber, or the soil or water in which the plants are growing, is effective to protect the plants from attack by the fungus or pests.

The formula I fungicidal agents may be formulated as solutions, suspensions, emulsifiable concentrates, flowable concentrates, wettable powders and the like which are diluted with water or other suitable polar solvents, generally in situ, and then applied as a dilute spray.

Said formula I agents may also be formulated in dry compacted granules, dusts, dust concentrates, suspension concentrates, microemulsions and the like. All compositions which lend themselves to seed, soil, water or foliage applications and provide effective plant protection are suitable. Said compositions include the formula I compound admixed with an inert liquid or solid diluent.

For example, wettable powders, dusts, and dust concentrate formulations can be prepared by grinding and blending together about 25% to 85% by weight of the formula I thioalkylpyrrole and about 75% to 15% by weight of a solid diluent such as bentonite, diatomaceous earth, kaolin, attapulgite, or the like, 1% to 5% by weight of a dispersing agent such as sodium lignosulfonate, and about 1% to 5% by weight of a nonionic surfactant, such as octylphenoxy polyethoxy ethanol, nonylphenoxy polyethoxy ethanol or the like.

A typical flowable liquid can be prepared by admixing about 40% by weight of the active ingredient with about 2% by weight of a gelling agent such as bentonite, 3% by weight of a dispersing agent such as sodium lignosulfonate, 1% by weight of polyethyleneglycol, and 54% by weight of water.

A typical emulsifiable concentrate can be prepared by dissolving about 15% to 70% by weight of the active ingredient in about 85% to 30% by weight of a solvent such as isophorone, toluene, butyl cellosolve, methyl acetate, propylene glycol monomethyl ether, or the like and dispersing therein about 1% to 5% by weight of a nonionic surfactant such as an alkylphenoxy polyethoxy alcohol.

Application of the material is made by adding a predetermined quantity of formulated product, such as described above, to the desired volume of water or other suitable solvent, alone or in combination with other agronomic chemicals for simultaneous use.

Advantageously, the compounds of the invention may be used effectively to control fungus infestations in conjunction with, or in combination with, other biological chemicals, including, but not limited to, probenazole, anilazine, benalaxyl, phosdiphen, capatafol, carboxin, chlorothanil, dichlorophen, diethofencarb, dithianon, ethoxyquin, fenfuram, ferbam, flusulfamide, iprobenfos, mancozeb, myclobutanil, oxadixyl, prochloraz, and the like.

Compositions of the invention are also effective for protecting turf grass from attack by pests such as grubs, chinch bugs and the like. Applications, such as spray applications, of compositions of the invention are generally effective at rates which provide about 0.125 kg/ha to 4.0 kg/ha of the active 1-(substituted)-thioalkylpyrrole compound. Of course, higher rates of application of said pyrrole compounds may be used, if desired.

Advantageously, the compounds of the invention may be used effectively in conjunction with, or in combination with, other biological chemicals, including other insecticides, nematicides, acaricides and molluscicides such as phosphates, carbamates, pyrethroids, halobenzoylureas, arylpyrroles, nitropyrroles, pyrrole carbonitriles and the like.

A particularly effective method for the control of terrestrial gastropods is to proffer the compound of formula I in a bait formulation. The bait formulation can be widely varied, but in general may contain about 3% to 20% wt/wt, preferably about 5% to 10% wt/wt, of the active ingredient, about 40% to 50% wt/wt of a solid edible substance, about 5% to 10% wt/wt of a carbohydrate source and, the remainder of the formulation, about 30% to 50% of a consumable liquid such as water. The carbohydrate source may be selected from sugar, molasses, corn syrup and the like.

In addition to the above-said bait formulations and spray formulations, the 1-(substituted)thioalkylpyrrole compounds of the present invention may be formulated into granular compositions, flowable compositions, wettable powders, dusts, microemulsions, emulsifiable concentrates and the like. All compositions which lend themselves to soil, water and foliage application and provide effective plant protection are suitable. Compositions of the invention include the formula I compound admixed with an inert solid or liquid carrier.

Where compositions of the invention are to be employed in combination treatments with other pesticidal agents, the composition may be applied as an admixture of the components or may be applied sequentially.

For a more clear understanding of the invention, specific detailed examples of it are set forth below. These examples are merely illustrative, and are not to be understood as limiting the scope and underlying principles of the invention in any way. Unless otherwise noted, all parts are parts by weight.

$H^1NMR$ and $C^{13}NMR$ designate proton nuclear magnetic resonance and carbon 13 nuclear magnetic resonance, respectively.

Example 1

**Preparation of 4-bromo-2-(p-chlorophenyl)-1-[(imidazol-2-ylthio)methyl]-5-trifluoromethylpyrrole-3-carbonitrile hydrobromide**

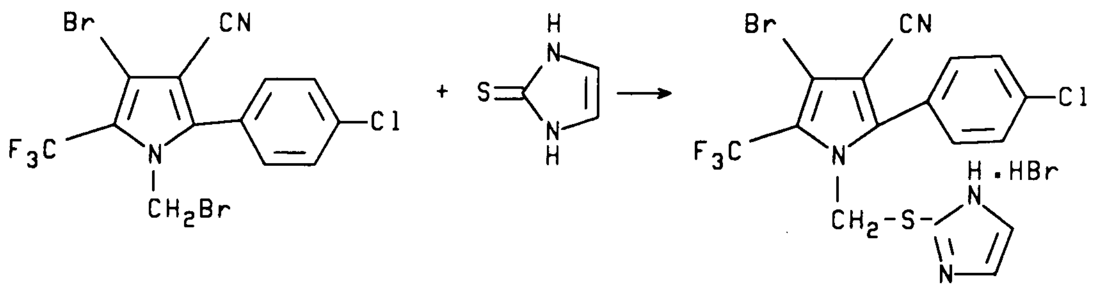

A mixture of 4-bromo-1-(bromomethyl)-2-(p-chlorophenyl)-5-(trifluoromethyl)pyrrole-3-carbonitrile (0.88g, 0.002 mol) and 4-imidazoline-2-thione (0.23g, 0.002 mol) in isopropanol is heated at reflux temperature for 3 hours, cooled to room temperature and filtered. The filtercake is washed with isopropanol and air-dried to give the title product as a white solid, 0.81 g (75% yield), mp 221°-223°C(dec).

Examples 2-20

Preparation of 1-[(substituted)thiomethyl]pyrroles

Using the procedure described in Example 1 and employing the appropriately substituted 1-(halomethyl)pyrrole substrate and a suitable thiourea reagent, the following 1-[(substituted)thiomethyl]pyrrole compounds shown in Table I are obtained.

# Table I

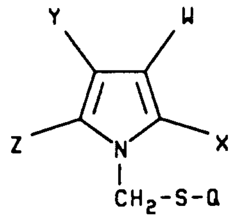
• acid salt

| Ex. No. | Z | Y | W | X | Q | acid salt | mp°C |
|---|---|---|---|---|---|---|---|
| 2 | $CF_3$ | Cl | CN | 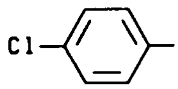 | 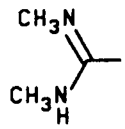 | HCl | 141-144 |
| 3 | $CF_3$ | Cl | CN | 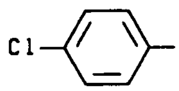 | 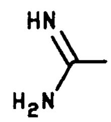 | HCl | 210-213 |
| 4 | $CF_3$ | Cl | CN | 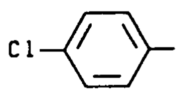 | 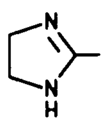 | HCl | 189-191 |
| 5 | $CF_3$ | Cl | CN | 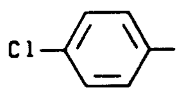 | 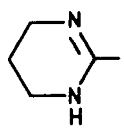 | HCl | 227-229 |
| 6 | $CF_3$ | Br | CN | 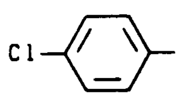 | 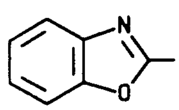 | free base | 135-137 |
| 7 | $CF_3$ | Br | CN | 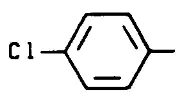 | 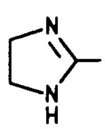 | HBr | 228-230 |
| 8 | $CF_3$ | Br | CN | 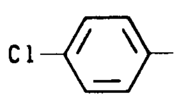 | 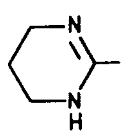 | HBr | )225 |

**Table I (cont.)**

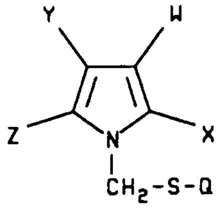

. acid salt

| Ex. No. | Z | Y | W | X | Q | acid salt | mp°C |
|---|---|---|---|---|---|---|---|
| 9 | $CF_3$ | Br | CN | 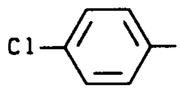 | 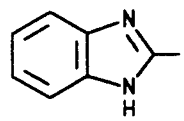 | HBr | 215-218 (dec) |
| 10 | $CF_3$ | Br | CN | 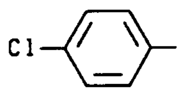 | 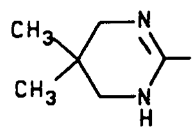 | HBr | 224-227 |
| 11 | $CF_3$ | Br | CN | 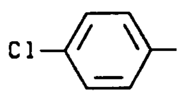 | 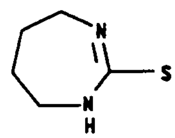 | HBr | 232-234 (dec) |
| 12 | $CF_3$ | Br | CN | 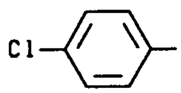 | 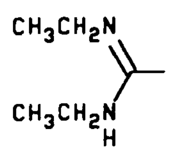 | HBr | 128-130 |
| 13 | Br | Br | CN | Br | 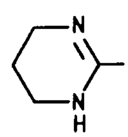 | HBr | 215 (dec) |
| 14 | Br | Br | CN | Br | 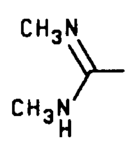 | HCl | 216 |
| 15 | Cl | Cl | CN | 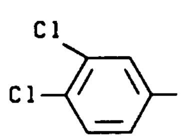 | 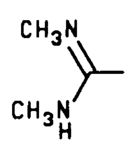 | HCl | 180-184 |

**Table I (cont.)**

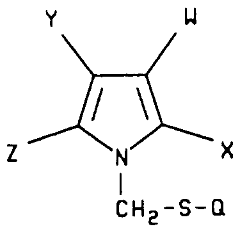

. acid salt

| Ex. No. | Z | Y | W | X | Q | acid salt | mp°C |
|---|---|---|---|---|---|---|---|
| 16 | $CF_3$ | Br | CN | 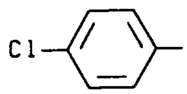 | 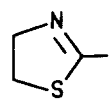 | free base | 124.5-125.5 |
| 17 | $CF_3$ | $CF_3$ | Br | 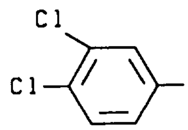 | 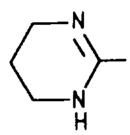 | HCl | 225-228 (dec) |
| 18 | $CF_3$ | $CF_3$ | Br | 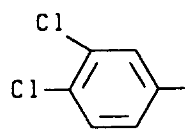 | 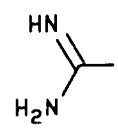 | HCl | 221 (dec) |
| 19 | Cl | CN | Cl | 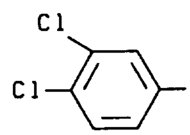 | 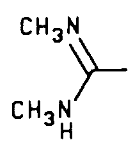 | HCl | 194-197 |
| 20 | Br | Br | CN | Br | 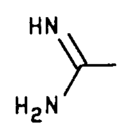 | HCl | 225 (dec) |

Ex. No. designates Example Number

Example 21

**Preparation of [3-Chloro-5-(p-chlorophenyl)-4-cyano-2-(trifluoromethyl)pyrrole-1-yl]dimethyldithiocarbamic acid, methyl ester**

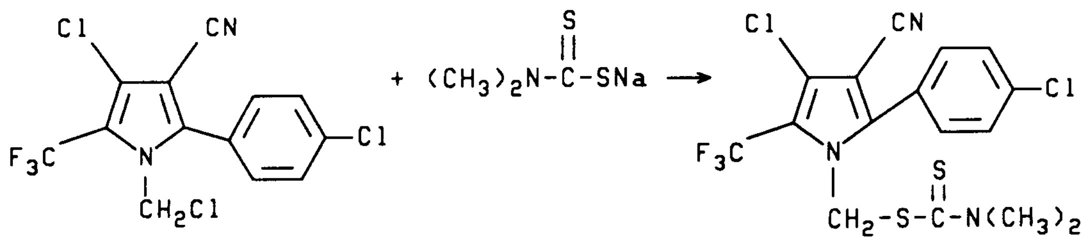

A mixture of 4-chloro-1-(chloromethyl)-2-(p-chlorophenyl)-5-(trifluoromethyl)pyrrole-3-carbonitrile (0.7g, 0.002 mol) and dimethyldithiocarbamic acid, sodium salt (0.57g, 0.004 mol) in dimethyl formamide is heated at 50°-55° for 30 minutes, cooled to room temperature and poured into water. The resultant mixture is extracted with ethyl acetate. The combined extracts are washed with a saturated sodium chloride solution, dried ($MgSO_4$) and concentrated in vacuo to give an orange oil residue. The residue is mixed with hot hexane, cooled and filtered to give an orange solid filtercake. Recrystallization of the solid from isopropanol gives the title product as a tan crystalline solid, 0.7g (80% yield), mp 129°-131°C.

Examples 22-43

Preparation of [(substituted pyrrole-1-yl)dithiocarbamate, thiocarbamate and thiophosphate methyl esters and thioalkyl derivatives

Using the procedure described in Example 21 and employing the appropriately substituted 1-halo methylpyrrole and the sodium salt of the desired dithiocarbamate, thiocarbamate, thiophosphate or mercaptate reagent the following compounds shown in Table II are obtained.

## Table II

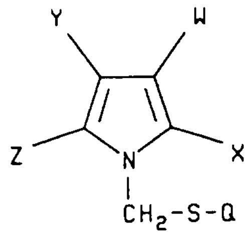

| Ex. No. | Z | Y | W | X | Q | mp°C |
|---|---|---|---|---|---|---|
| 22 | $CF_3$ | Cl | CN | 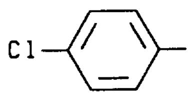 | S<br>‖<br>$(CH_3)_2CHOC-$ | 94-95 |
| 23 | $CF_3$ | Cl | CN | 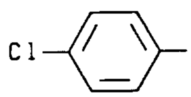 | S<br>‖<br>$CH_3CH_2OC-$ | (oil) |
| 24 | $CF_3$ | Br | CN | 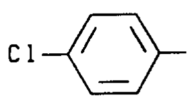 | S<br>‖<br>$(CH_3)_2NC-$ | 125-128 |
| 25 | $CF_3$ | Br | CN | 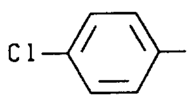 | $CH_3$ | |
| 26 | $CF_3$ | Br | CN | 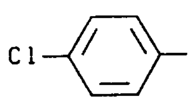 | S<br>‖<br>$(CH_3CH_2)_2NC-$ | 101-103 |

**Table II (cont.)**

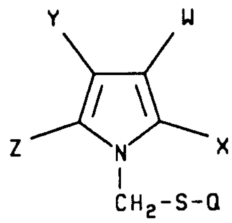

| Ex. No. | Z | Y | W | X | Q | mp°C |
|---|---|---|---|---|---|---|
| 27 | $CF_3$ | Br | CN | 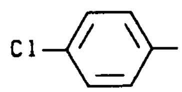 | $[(CH_3)_2CHO]_2P(=S)-$ | 108-110 |
| 28 | Cl | Cl | CN | 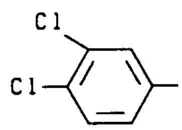 | $CH_3CH_2OC(=S)-$ | 82-85 |
| 29 | $CF_3$ | Br | CN | 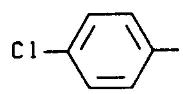 | $CH_3CH_2OC(=S)-$ | 85-86 |
| 30 | $CF_3$ | Br | CN | 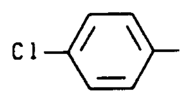 | $(CH_3)_2CHOC(=S)-$ | 120-121.5 |
| 31 | Br | Br | CN | Br | $(CH_3)_2NC(=S)-$ | 170-172 |
| 32 | Cl | Cl | CN | 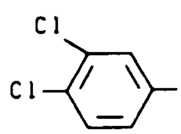 | $(CH_3)_2NC(=S)-$ | 194-197 |
| 33 | $CF_3$ | $CF_3$ | Br | 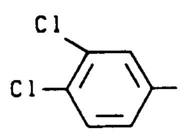 | $(CH_3)_2NC(=S)-$ | 128-129 |

**Table II (cont.)**

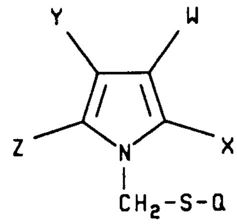

| Ex. No. | Z | Y | W | X | Q | mp°C |
|---|---|---|---|---|---|---|
| 34 | Br | Br | CN | Br | 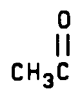 | 135-137 |
| 35 | $CF_3$ | $CF_3$ | Br | 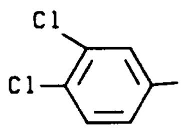 | 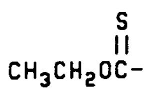 | (oil) |
| 36 | Cl | CN | Cl | 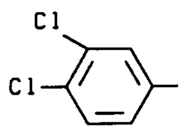 | 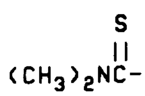 | 120-123 |
| 37 | Cl | CN | Cl | 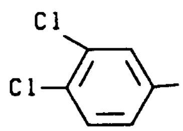 | 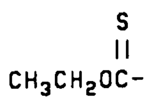 | 100-105 |
| 38 | Br | Br | CN | Br | CN | 168-170 |
| 39 | Br | Br | CN | 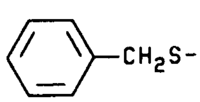 | 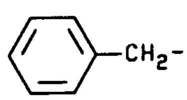 | 50-53 |
| 40 | Br | Br | CN | Br | 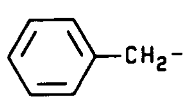 | 106-110.5 |

## Table II (cont.)

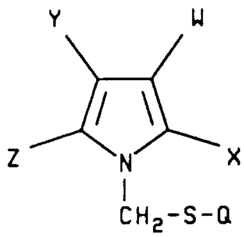

| Ex. No. | Z | Y | W | X | Q | mp°C |
|---|---|---|---|---|---|---|
| 41 | $CF_3$ | Br | CN | 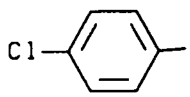 | 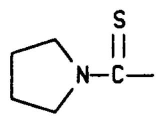 | 106-110 |
| 42 | $CF_3$ | Br | CN | 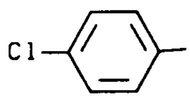 | CN | 95-97 |
| 43 | Br | Br | CN | $CH_3S$- | $CH_3$- | 110-111 |

Ex. No. designates Example Number

Example 44

### Preparation of 4,5-dibromo-1[(methylthio)methyl]-2-(α,α,α-trifluoro-p-tolyl)pyrrole-3-carbonitrile

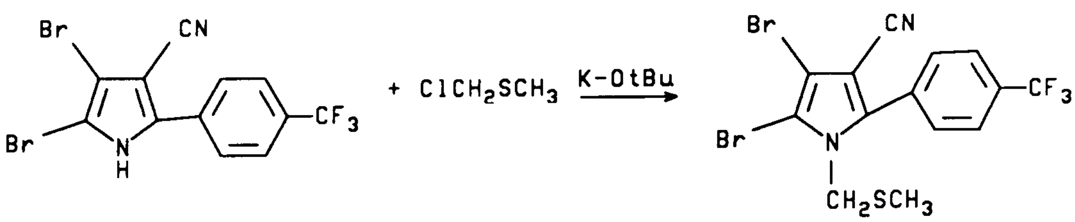


A solution of 4,5-dibromo-2-(α,α,α-trifluoro-p-tolyl)pyrrole-3-carbonitrile (0.56g, 1.42 mmol) in dry tetrahydrofuran, under nitrogen, is treated portion-wise with potassium t-butoxide (0.21g, 1.81 mmol), stirred at 25°C for 3/4 hour, treated dropwise with chloromethyl methyl thioether (0.18g, 1.81 mmol), stirred at room temperature for 16 hours, heated at 49°-60°C until reaction is complete by thin layer chromatography. The reaction mixture is diluted with water and ether. The phases are separated and the organic layer

is dried over $MgSO_4$ and concentrated in vacuo to give a black oil residue. The residue is flash chromatographed (silica/ 100:100:1 ether:petroleum ether:ethyl acetate) to give the title product as a red solid, mp 140°-145°C, identified by $H^1NMR$ and mass spectral analyses.

Example 45

**Preparation of 4,5-dibromo-1[(phenylthio)methyl]-2-(α,α,α-trifluoro-p-tolyl)pyrrole-3-carbonitrile**

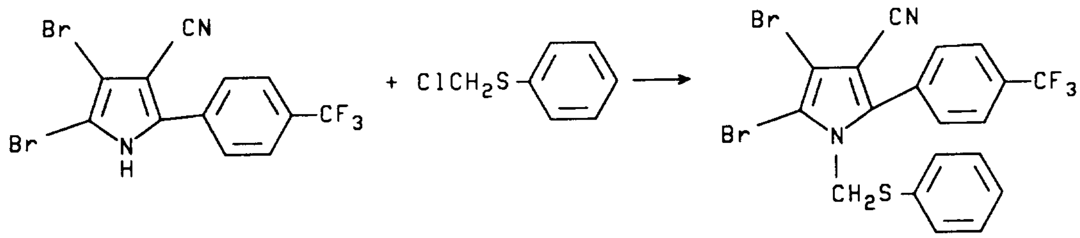

Following the procedure described in Example 44, above, and substituting chloromethyl phenyl thioether as the alkylating reagent, the title product is obtained as a red oil, bp >200°C/0.7mmHg, identified by $H^1NMR$, $C^{13}NMR$ and mass spectral analyses.

Example 46

**Preparation of 4-bromo-2-(p-chlorophenyl)-1-[(phenylsulfonyl)methyl]-5-trifluoromethyl)pyrrole-3-carbonitrile**

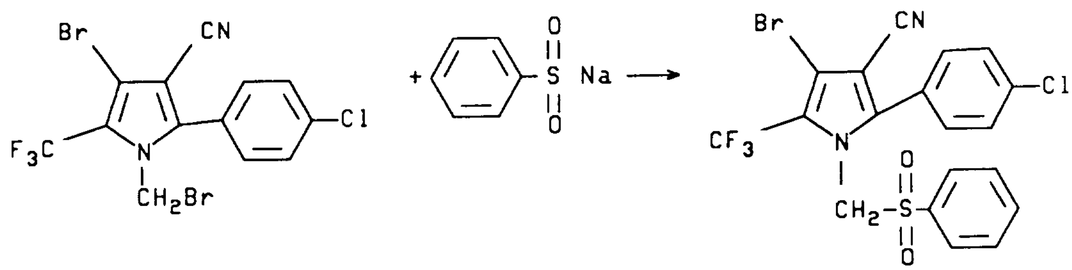

A stirred solution of 4-bromo-1-(bromomethyl)-2-(p-chlorophenyl)-5-(trifluoromethyl)pyrrole-3-carbonitrile (0.65 g, 1.5 mmol) in dry dimethyl formamide is treated with sodium benzylsulfonate (0.485 g, 3.0 mmol), heated at 70°C for 3 hours, cooled to room temperature and diluted with a mixture of water and ethyl acetate. The organic phase is separated, dried over $MgSO_4$ and concentrated in vacuo to give a yellow oil residue. Column chromatography (silica gel/4:1 hexanes:ethyl acetate) gives the title product as a white solid, mp 165°-166.5°C, identified by $H^1NMR$ and mass spectral analyses.

EXAMPLE 47

Evaluation of *in vivo* fungicidal activity of test compounds

Test compounds are dissolved in acetone and diluted with deionized water containing about 0.05% TWEEN 20®, a polyoxyethylene sorbitan monolaurate surfactant manufactured by Atlas Chemical Industries, to give a concentration of 400 ppm. Subsequent dilutions are made with an 0.05% aqueous solution of TWEEN 20®.

Host plants are sprayed with test solution, dried and inoculated with fungi. When disease symptom development is optimal, the plants are rated for disease control according to the rating scale shown below. Each test contains inoculated treated plants, inoculated untreated plants and a reference standard.

| Symbol | Disease | Pathogen |
|---|---|---|
| AS | Apple Scab | *Venturia inaequalis* |
| GDM | Grape Downy Mildew | *Plasmopara viticola* |
| SBC | Sugarbeet Cercospora | *Cercospora beticola* |
| WLR | Wheat Leaf Rrust | *Puccinia recondita, tritici* |
| WPM | Wheat Powdery Mildew | *Erysiphe graminis, tritici* |

| Rating Scale | |
|---|---|
| Rating | Range % Control |
| 0 | 0 |
| 1 | 1 - 19 |
| 2 | 20 - 29 |
| 3 | 30 - 39 |
| 4 | 40 - 59 |
| 5 | 60 - 74 |
| 6 | 75 - 89 |
| 7 | 90 - 96 |
| 8 | 97 - 99 |
| 9 | 100 |
| - | no evaluation |

When more than one test is run, the data are averaged. The data obtained are shown in Table III.

Table III

| Evaluation of Test Compounds For The Control of Plant Pathogenic Fungi | | | | | | |
|---|---|---|---|---|---|---|
| Compound | Rate (ppm) | Disease Control | | | | |
| | | AS | GDM | SBC | WLR | WPM |
| 4,5-dibromo -1-[(phenylthio)methyl]-2-(alpha, alpha,alpha-trifluoro-p-tolyl)pyrrole-3-carbonitrile | 25 | - | - | 6.0 | - | - |
| | 100 | - | - | 7.0 | - | - |
| | 400 | 0 | 0 | 7.5 | 6.0 | 1.0 |
| 1-[(cyanothio)methyl]-2,4,5-tribromopyrrole-3-carbonitrile | 400 | 2.0 | 3.0 | 0 | 5.0 | 0 |
| O,O-diisopropyl S-(2,3,5-tribromo-4-cyanopyrrole-1-yl)methyl ester phosphorodithioic acid | 25 | - | - | - | - | 0 |
| | 100 | - | - | - | - | 4.0 |
| | 400 | 0 | 0 | 0 | 0 | 7.0 |

**EXAMPLE 48**

Evaluation of *in vitro* fungicidal activity of test compounds

Test compounds are dissolved in acetone, diluted with sterile, deionized water and mixed with a partially cooled, autoclaved, chemically defined agar medium. This mixture is poured into several 100mm x 15mm sterile plastic plates and allowed to solidify. The surface of each plate is inoculated with a single disc of mycelium and agar cut with an 8mm diameter cork borer from actively growing cultures of assay fungi on a chemically defined agar medium. The plates are incubated at room temperature. Growth inhibition is determined when the mycelial growth on unamended control media has reached the edge of the petri plates. The time interval varies depending on the growth rate of the fungus; two days for *Pythium ultimum* - (pyth) and *Rhizoctonia solani* (Rhiz) , five days for *Botrytis cinerea* (Betry) and 7-10 days for *Pseudocercosporella herpotrichoides* (Pseudo). Each colony diameter is measured and compared with the untreated control and the percent inhibition is calculated as follows:

$$\%\ \text{Mycelial growth inhibition} = \frac{\text{Growth of untreated control (mm) minus the Growth of treatment (mm)}}{\text{Growth of untreated control (mm)}} \times 100$$

Each test includes a solvent blank consisting of the formulation without a test compound in addition to the untreated control.

Assay fungi include the plant pathogens, *Pythium ultimum* (Pyth) and *Rhizoctonia solani* (Rhiz).

When more than one test is run the data are averaged. The data obtained are shown in Table IV.

Table IV

| Evaluation Of Test Compounds For The Control Of The Mycelial Growth of Fungi | | | |
|---|---|---|---|
| Compound Name | Rate (ppm) | % Growth Inhibition | |
| | | Pyth | Rhiz |
| 4,5-dibromo-1-[(phenylthio)methyl]-2-(alpha,alpha,alphatrifluoro-p-tolyl)pyrrole-3-carbonitrile | 1 | 0 | 0 |
| | 10 | 7.0 | 3.5 |
| | 25 | 4.5 | 2.0 |
| 1-[(cyanothio)methyl]-2,4,5-tribromopyrrole-3-carbonitrile | 1 | 1.5 | 1.5 |
| | 10 | 4 | 3.5 |
| | 25 | 4.5 | 5.8 |

Example 49

Insecticidal and acaricidal evaluations of test compounds

Test solutions are prepared by dissolving the test compound in a 35% acetone in water mixture to give a concentration of 10,000 ppm. Subsequent dilutions are made with water as needed.

*Heliothis virescens*, egg, tobacco budworm

A young cotton leaf about 6-7cm in length is dipped in an agitated test suspension for about 3 seconds. Eggs are collected on a cheesecloth and the cloth is cut into 10-20mm squares, each containing approximately 50-100 eggs which are 6-30 hours old. The egg-containing cheesecloth square is similarly dipped in a test suspension and placed on a treated cotton leaf. The combination is dried in a hood and then placed in an 8 ounce Dixie® cup #2168-ST (240ml volume, 6cm tall, 9.5cm top diameter, 8.0cm bottom diameter) into which a 5cm length of damp dental wick has been placed. The cup is covered with a clear plastic lid and held for 3 days, after which mortality counts are made.

*Heliothis virescens*, 3rd instar tobacco budworm

Cotton cotyledons are dipped in the test solution and allowed to dry in a hood. When dry, each is cut into quarters and ten sections are placed individually in 30 mL plastic medicine cups containing a 5-7mm long piece of damp dental wick. One 3rd instar caterpillar is added to each cup and the cup is sealed with MYLAR® film. Treatments are maintained for 3 days before a mortality count and observation of reduction in feeding damage are made.

*Spodoptera eridania*, 3rd instar larvae, southern armyworm

A Sieva limabean leaf expanded to 7-8 cm in length is dipped in the test solution with agitation for 3 seconds and allowed to dry in a hood. The leaf is then placed in a 100 x 10 mm petri dish containing a damp filterpaper on the bottom and ten 3rd instar caterpillars. At 3 and 5 days, observations are made of mortality, reduced feeding, or any interference with normal molting.

*Spodoptera eridania*, 3rd instar larvae, southern armyworm, 7-day residual

The limabean leaves treated in the above test are maintained under high intensity lamps in the greenhouse for 7 days. These lamps duplicate the effects of a bright sunny day in June in New Jersey and are kept on for 14 hour day length. After 7 days, the foliage is sampled and assayed as in the above-said test.

*Spodoptera eridania*, systemic uptake, 3rd instar larvae, southern armyworm

An emulsion containing 0.1 gm of the test compound, 0.2 gm of Emulphor EL-620® emulsifier, 10 mL of acetone and 90 mL of water is prepared and diluted 10-fold with water to give a 100 ppm emulsion. Subsequent 10-fold dilutions are made with water as needed. Sieva limabean plants, with the primary leaves expanded to a length of 7-8 cm, are cut off at least 3 cm above the soil level to avoid contamination with soil bacteria which will cause decay of the stem during the test. The cut stem is placed in the test emulsion and wrapped with a bit of cotton to hold the stem off the bottom of the bottle and to limit evaporation. The treated stem is thus maintained for 3 days at 27° C to allow the test compound to be taken up into the plant. Following this, one leaf is removed from the plant and placed in a 100 x 10 mm petri dish with 10 southern armyworms as described above. Mortality counts and observations of feeding damage are made 3 and 5 days later.

*Diabrotic undecimpunctata howardi*, 3rd instar southern corn rootworm

One cc of fine talc is placed in a 30 mL wide-mouth screw-top glass jar. One mL of the appropriate acetone test solution is pipetted onto the talc so as to provide 1.25 and 0.25 mg of active ingredient per jar. The jars are set under a gentle air flow until the acetone is evaporated. The dried talc is loosened, 1 cc of millet seed is added to serve as food for the insects and 25 mL of moist soil is added to each jar. The jar is capped and the contents thoroughly mixed on a Vortex Mixer. Following this, ten 3rd instar rootworms are added to each jar and the jars are loosely capped to allow air exchange for the larvae. The treatments are held for 6 days when mortality counts are made. Missing larvae are presumed dead, since they decompose rapidly and can not be found. The concentrations used in this test correspond approximately to 50 and 10 kg/ha, respectively.

*Tetranychus urticae* (OP-resistant strain), 2-spotted spider mite

Sieva limabean plants with primary leaves expanded to 7-8 cm are selected and cut back to one plant per pot. A small piece is cut from an infested leaf taken from the main colony and placed on each leaf of the test plants. This is done about 2 hours before treatment to allow the mites to move over to the test plant and to lay eggs. The size of the cut, infested leaf is varied to obtain about 100 mites per leaf. At the time of test treatment, the piece of leaf used to transfer the mites is removed and discarded. The newly mite-infested plants are dipped in the test solution for 3 seconds with agitation and set in the hood to dry. After 2 days one leaf is removed and mortality counts are made. After 5 days, another leaf is removed and observations are made of mortality of the eggs and/or newly emerged nymphs.

*Aphis fabae*, mixed instar, bean aphid

Pots containing single nasturtium plants (*Tropaeolum sp.*) about 5 cm tall are infested with about 100-200 aphids one day prior to treatment. Each pot is sprayed with the test solution for 2 revolutions of a 4 rpm turntable in a hood, using a #154 DeVilbiss atomizer. The spray tip is held about 15 cm from the plant and the spray directed so as to give complete coverage of the plants and the aphids. The sprayed pots are set on their sides on white enamel trays and mortality estimates are made after 2 days.

*Empoasca abrupta*, adult, western potato leafhopper

A Sieva limabean leaf about 5 cm long is dipped in the test solution for 3 seconds with agitation and placed in a hood to dry. The treated leaf is placed in a 100 x 10 mm petri dish containing a moist filter paper and about 10 adult leafhoppers are added. After 2 days, mortality counts are made.

*Empoasca abrupta*, adult, western potato leafhopper Systemic Uptake

An emulsion containing 0.1 gm of the test material, 0.2 gm of Emulphor EL-620® emulsifier, 10 mL of acetone and 90 mL of water is prepared and diluted 10-fold with water to give a 100 ppm emulsion. Subsequent 10-fold dilutions are made with water as needed. Sieva limabean plants, with the primary leaves expanded to a length of 7-8 cm, are cut off at least 3 cm above the soil level to avoid contamination with soil bacteria which will cause decay of the stem during the test. The cut stem is placed in the test emulsion and wrapped with a bit of cotton to hold the stem off the bottom of the bottle and to limit evaporation. The

treated stem is maintained for 3 days at 27°C to allow the test compound to be taken up into the plant. Following this, one leaf is removed from the plant and placed in a 100 x 10 mm petri dish containing a moist filter paper and about 10 adult leafhoppers as described above. Mortality counts are made after 2 days.

All of the above tests are rated according to the rating scale shown below.

| Rating Scale: | |
|---|---|
| 0 = no effect | 5 = 56-65% kill |
| 1 = 10-25% kill | 6 = 66-75% kill |
| 2 = 26-35% kill | 7 = 76-85% kill |
| 3 = 36-45% kill | 8 = 86-99% kill |
| 4 = 46-55% kill | 9 = 100% kill |

The data obtained are shown in Tables V and VI wherein the test compounds are identifed by Example Number.

**TABLE V**

Insecticidal And Acaricidal Evaluation Of Test Compounds

| Compound | TOBACCO BUDWORM | | | | | | ARMYWORM | | | | | | | | | SOUTHERN CORN ROOTWORM | | |
|---|---|---|---|---|---|---|---|---|---|---|---|---|---|---|---|---|---|---|
| | Egg (ppm) | | | 3rd Instar (ppm) | | | Day 3 (ppm) | | | | Day 5 (ppm) | | | | Stem Sys (ppm) | (ppm) | | |
| (Ex. No.) | 1000 | 300 | 100 | 1000 | 100 | 10 | 1000 | 100 | 10 | R | 1000 | 100 | 10 | R | 1000 | 50 | 10 | 1 |
| 44 | 0 | - | - | 9 | 0 | 0 | 9 | 9 | 0 | 0 | 9 | 9 | 0 | 2 | 0 | 3 | 0 | - |
| 45 | 0 | - | - | 0 | 0 | - | 8 | 0 | - | 0 | 8 | 0 | - | 0 | 0 | 0 | - | - |
| 2 | 0 | - | 0 | 9 | 9 | 9 | 9 | 9 | 9 | 9 | 9 | 9 | 9 | 9 | 0 | 8 | 0 | 0 |
| 3 | 5 | - | 0 | 9 | 9 | 8 | 9 | 9 | 8 | - | 9 | 9 | 9 | - | 0 | 9 | 0 | 0 |
| 22 | 0 | - | - | 9 | 9 | 3 | 9 | 9 | 9 | - | 9 | 9 | 9 | - | 0 | 9 | 8 | 3 |
| 23 | 0 | - | - | 9 | 9 | 5 | 9 | 9 | 9 | - | 9 | 9 | 9 | - | - | 9 | 6 | 0 |
| 4 | 9 | - | 9 | 9 | 9 | 9 | 9 | 9 | 9 | 9 | 9 | 9 | 9 | 9 | 0 | 0 | - | - |
| 25 | 0 | - | - | 9 | 9 | 8 | 9 | 9 | 0 | 9 | 9 | 9 | 0 | 9 | 0 | 0 | 0 | - |
| 5 | 0 | - | - | 9 | 9 | 0 | 9 | 9 | 4 | 9 | 9 | 9 | 4 | 9 | 8 | 0 | - | - |
| 21 | 0 | - | 0 | 9 | 9 | 9 | 9 | 9 | 9 | 9 | 9 | 9 | 9 | 9 | 0 | 9 | 0 | 0 |
| 24 | 0 | - | - | 9 | 9 | 0 | 9 | 9 | 9 | 9 | 9 | 9 | 9 | 9 | 0 | 9 | 0 | 0 |
| 46 | 0 | - | - | 9 | 4 | - | 9 | 0 | 0 | 4 | 9 | 0 | 0 | 9 | 0 | 8 | 0 | 0 |

**TABLE V (Cont.)**

**Insecticidal And Acaricidal Evaluation Of Test Compounds**

| Compound | TOBACCO BUDWORM | | | | | | ARMYWORM | | | | | | | | | SOUTHERN CORN ROOTWORM | | |
|---|---|---|---|---|---|---|---|---|---|---|---|---|---|---|---|---|---|---|
| | Egg (ppm) | | | 3rd Instar (ppm) | | | Day 3 (ppm) | | | | Day 5 (ppm) | | | | Stem Sys (ppm) | (ppm) | | |
| (Ex. No.) | 1000 | 300 | 100 | 1000 | 100 | 10 | 1000 | 100 | 10 | R | 1000 | 100 | 10 | R | 1000 | 50 | 10 | 1 |
| 6 | 0 | - | - | 9 | 8 | 0 | 9 | 9 | 0 | 9 | 9 | 9 | 5 | 9 | 0 | 9 | 5 | 0 |
| 7 | 9 | 8 | 0 | 9 | 9 | 5 | 9 | 9 | 9 | 9 | 9 | 9 | 9 | 9 | 0 | 9 | 0 | 0 |
| 8 | 0 | - | 0 | 9 | 9 | 3 | 9 | 9 | 0 | 9 | 9 | 9 | 6 | 9 | 9 | 0 | - | - |
| 9 | 0 | - | - | 0 | 0 | - | 6 | 0 | - | 9 | 7 | 0 | - | 9 | 0 | 0 | - | - |
| 10 | 0 | - | 0 | 9 | 8 | 0 | 9 | 9 | 0 | 9 | 9 | 9 | 0 | 9 | 0 | 0 | - | - |
| 11 | 5 | - | 3 | 9 | 9 | 6 | 9 | 9 | 9 | 9 | 9 | 9 | 9 | 9 | 9 | 0 | - | - |
| 12 | 0 | - | - | 9 | 9 | 8 | 9 | 9 | 9 | 9 | 9 | 9 | 9 | 9 | 0 | 8 | 9 | 0 |
| 26 | 0 | - | - | 9 | 9 | 9 | 9 | 9 | 9 | 9 | 9 | 9 | 9 | 9 | 0 | 9 | 4 | 0 |
| 27 | 0 | - | - | 9 | 7 | 0 | 9 | 9 | 0 | 9 | 9 | 9 | 0 | 9 | 0 | 9 | 7 | 6 |
| 1 | 0 | - | 0 | 9 | 9 | 4 | 9 | 9 | 9 | 9 | 9 | 9 | 9 | 9 | 0 | 0 | - | - |
| 28 | 0 | - | 0 | 9 | 3 | 0 | 9 | 9 | 6 | 9 | 9 | 9 | 6 | 9 | 0 | 0 | - | - |
| 29 | 4 | - | 0 | 9 | 9 | 4 | 9 | 9 | 9 | 9 | 9 | 9 | 9 | 9 | 0 | 9 | - | - |

**TABLE V (Cont.)**

**Insecticidal And Acaricidal Evaluation Of Test Compounds**

| Compound | TOBACCO BUDWORM | | | | | | ARMYWORM | | | | | | | | | SOUTHERN CORN ROOTWORM | | |
|---|---|---|---|---|---|---|---|---|---|---|---|---|---|---|---|---|---|---|
| | Egg (ppm) | | | 3rd Instar (ppm) | | | Day 3 (ppm) | | | | Day 5 (ppm) | | | | Stem Sys (ppm) | (ppm) | | |
| (Ex. No.) | 1000 | 300 | 100 | 1000 | 100 | 10 | 1000 | 100 | 10 | R | 1000 | 100 | 10 | R | 1000 | 50 | 10 | 1 |
| 30 | 0 | - | 0 | 9 | 9 | 4 | 9 | 9 | 9 | 9 | 9 | 9 | 9 | 9 | 0 | 9 | - | - |
| 13 | 0 | - | - | 0 | - | - | 9 | 0 | - | 0 | 9 | 3 | - | 7 | 7 | 0 | - | - |
| 31 | 0 | - | - | 6 | - | - | 9 | 9 | 0 | 9 | 9 | 9 | 0 | 9 | 0 | 0 | - | - |
| 14 | 0 | - | - | 8 | 0 | - | 9 | 9 | 0 | 0 | 9 | 9 | 0 | 5 | 0 | 0 | - | - |
| 15 | 0 | - | 0 | 9 | 6 | 0 | 9 | 9 | 9 | 9 | 9 | 9 | 9 | 9 | 0 | 0 | - | - |
| 32 | 0 | - | - | 0 | 0 | - | 9 | 3 | 0 | 0 | 9 | 3 | 0 | 0 | 0 | 0 | - | - |
| 16 | 0 | - | 0 | 9 | 9 | 9 | 9 | 9 | 9 | 9 | 9 | 9 | 9 | 9 | 4 | 9 | - | - |
| 17 | 0 | - | - | 9 | 8 | 8 | 9 | 9 | 9 | 9 | 9 | 9 | 9 | 9 | 0 | 7 | - | - |
| 18 | 0 | - | - | 9 | 9 | 0 | 9 | 9 | 0 | 9 | 9 | 9 | 0 | 9 | 0 | 0 | - | - |
| 33 | 0 | - | - | 9 | 9 | 7 | 9 | 9 | 9 | 9 | 9 | 9 | 9 | 9 | 0 | - | - | - |
| 43 | 0 | - | - | 0 | - | - | 9 | 0 | 0 | * | 9 | 0 | 0 | * | 0 | 0 | - | - |
| 34 | 8 | - | - | 9 | 0 | - | 9 | 9 | 5 | * | 9 | 9 | 5 | * | 0 | 0 | - | - |

**TABLE V (Cont.)**

**Insecticidal And Acaricidal Evaluation Of Test Compounds**

| Compound | TOBACCO BUDWORM | | | | | | ARMYWORM | | | | | | | | | SOUTHERN CORN ROOTWORM | | |
|---|---|---|---|---|---|---|---|---|---|---|---|---|---|---|---|---|---|---|
| | Egg (ppm) | | | 3rd Instar (ppm) | | | Day 3 (ppm) | | | | Day 5 (ppm) | | | | Stem Sys (ppm) | (ppm) | | |
| (Ex. No.) | 1000 | 300 | 100 | 1000 | 100 | 10 | 1000 | 100 | 10 | R | 1000 | 100 | 10 | R | 1000 | 50 | 10 | 1 |
| 35 | 0 | - | - | 9 | 9 | 8 | 9 | 9 | 9 | 9 | 9 | 9 | 9 | 9 | 0 | 0 | - | - |
| 36 | 0 | - | - | 9 | 9 | 0 | 9 | 9 | 8 | 9 | 9 | 9 | 8 | 9 | 0 | 0 | - | - |
| 19 | 0 | - | - | 9 | 9 | 0 | 9 | 9 | 0 | 9 | 9 | 9 | 0 | 9 | 0 | 0 | - | - |
| 37 | 0 | - | - | 9 | 9 | 0 | 9 | 9 | 9 | 9 | 9 | 9 | 9 | 9 | 0 | 0 | - | - |
| 38 | - | - | - | - | - | - | 9 | - | - | - | 9 | - | - | - | - | 0 | - | - |
| 20 | 0 | 0 | 0 | 9 | 0 | 0 | 9 | 9 | 0 | 6 | 9 | 9 | 0 | * | - | 0 | - | - |
| 40 | 0 | - | - | 0 | 0 | - | 9 | 0 | 0 | 0 | 9 | 0 | 0 | * | 0 | 0 | - | - |

R designates Residual
Stem Sys designates Stem Systemic

**TABLE VI**

**Insecticidal And Acaricidal Evaluation Of Test Compounds**

| Compound | OP-RESISTANT MITES | | | | | | | | | APHIDS | LEAFHOPPER | | |
|---|---|---|---|---|---|---|---|---|---|---|---|---|---|
| | Adult (ppm) | | | Egg (ppm) | | | Nymph (ppm) | | | (ppm) | Contact (ppm) | | Systemic (ppm) |
| (Ex. No.) | 300 | 100 | 10 | 300 | 100 | 10 | 300 | 100 | 10 | 100 | 100 | 10 | 100 |
| 44 | 9 | 7 | 0 | 0 | 7 | 0 | 8 | 0 | 5 | 0 | 7 | 0 | 0 |
| 2 | 9 | 9 | 8 | 0 | 9 | 0 | 9 | - | 7 | 0 | 9 | 8 | 0 |
| 3 | 4 | 9 | 8 | 4 | 9 | 1 | 9 | - | 0 | 0 | 9 | 9 | 0 |
| 22 | 9 | 9 | 9 | 0 | 9 | 9 | 9 | - | - | 7 | 9 | 9 | 0 |
| 23 | 9 | 9 | 9 | 0 | 9 | 9 | 9 | - | - | 5 | 9 | 9 | 0 |
| 4 | 9 | 6 | 0 | 9 | 0 | 0 | - | 0 | 0 | 0 | 9 | 0 | 0 |
| 25 | 7 | - | - | 0 | - | - | 0 | - | - | 0 | 9 | 0 | 0 |
| 5 | 9 | 8 | 0 | 0 | 9 | 0 | 0 | - | 0 | 0 | 9 | 4 | 8 |
| 21 | 9 | 9 | 9 | 0 | 8 | 0 | 9 | 0 | 0 | 0 | 9 | 5 | 0 |
| 24 | 0 | 9 | 9 | 9 | 9 | 0 | - | - | 0 | 7 | 9 | 0 | 0 |
| 7 | 9 | 9 | 9 | 0 | 9 | 0 | 0 | - | 0 | 0 | 9 | 5 | 0 |
| 8 | 9 | 9 | 0 | 0 | 8 | 4 | 0 | 8 | 0 | 0 | 8 | 0 | - |

**TABLE VI (Cont.)**

Insecticidal And Acaricidal Evaluation Of Test Compounds

| | OP-RESISTANT MITES | | | | | | | | | APHIDS | LEAFHOPPER | | |
|---|---|---|---|---|---|---|---|---|---|---|---|---|---|
| | Adult (ppm) | | | Egg (ppm) | | | Nymph (ppm) | | | (ppm) | Contact (ppm) | | Systemic (ppm) |
| Compound (Ex. No.) | 300 | 100 | 10 | 300 | 100 | 10 | 300 | 100 | 10 | 100 | 100 | 10 | 100 |
| 10 | 0 | - | - | 0 | - | - | 0 | - | - | 0 | 7 | 0 | 6 |
| 11 | 0 | - | - | 0 | - | - | 0 | - | - | 0 | 9 | 8 | 9 |
| 12 | 0 | - | - | 0 | - | - | 0 | - | - | 0 | 9 | 5 | - |
| 1 | 0 | - | - | 0 | - | - | 0 | - | - | 0 | 9 | 7 | 0 |
| 28 | 9 | 9 | 3 | 0 | 9 | 0 | 0 | - | 3 | 0 | 9 | 0 | 0 |
| 29 | 9 | 9 | 9 | 0 | 9 | 9 | 0 | - | - | 0 | 9 | 9 | 0 |
| 30 | 9 | 9 | 9 | 0 | 9 | 9 | 0 | - | - | 3 | 9 | 8 | 0 |
| 13 | 5 | - | - | 0 | - | - | 0 | - | - | 0 | 0 | - | 0 |
| 14 | 0 | - | - | 0 | - | - | 0 | - | - | 0 | 0 | - | 7 |
| 15 | 0 | - | - | 0 | - | - | 0 | - | - | 0 | 6 | 0 | 0 |
| 16 | 0 | - | - | 0 | - | - | 0 | - | - | 8 | 9 | 9 | 0 |
| 17 | 7 | 0 | 0 | 0 | 7 | 0 | 0 | 0 | 0 | 0 | 5 | - | 0 |

**TABLE VI (Cont.)**

**Insecticidal And Acaricidal Evaluation Of Test Compounds**

| Compound | OP-RESISTANT MITES Adult (ppm) | | | OP-RESISTANT MITES Egg (ppm) | | | OP-RESISTANT MITES Nymph (ppm) | | | APHIDS (ppm) | LEAFHOPPER Contact (ppm) | | LEAFHOPPER Systemic (ppm) |
|---|---|---|---|---|---|---|---|---|---|---|---|---|---|
| (Ex. No.) | 300 | 100 | 10 | 300 | 100 | 10 | 300 | 100 | 10 | 100 | 100 | 10 | 100 |
| 18 | 9 | 9 | 0 | 0 | 0 | 0 | 0 | 0 | 0 | 0 | 9 | 4 | 0 |
| 33 | 7 | 0 | 0 | 0 | 8 | 0 | 0 | 0 | 0 | 0 | 8 | 0 | 0 |
| 34 | 0 | - | - | 0 | - | - | 0 | - | - | 8 | 0 | - | 0 |
| 35 | 9 | 9 | 8 | 0 | 9 | 3 | 0 | - | 0 | 0 | 9 | 9 | 0 |
| 36 | 7 | 0 | 0 | 0 | 0 | 0 | 0 | 0 | 0 | 0 | 6 | - | 0 |
| 19 | 5 | - | - | 0 | - | - | 0 | - | - | 0 | 9 | 0 | 0 |
| 37 | 7 | 9 | 0 | 0 | 9 | 2 | 0 | - | 4 | 0 | 6 | - | 0 |

## Claims

**1.** A compound having the structure

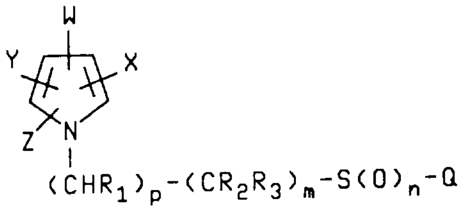

I

wherein

| | |
|---|---|
| W is | CN or $NO_2$; |
| X is | halogen or phenyl optionally substituted with one to three $C_1$-$C_3$alkyl, $C_1$-$C_3$alkoxy, $C_1$-$C_3$alkylthio, $C_1$-$C_3$alkylsulfinyl, $C_1$-$C_3$alkylsulfonyl, halogen, CN, $NO_2$, $CF_3$, $R_4CF_2B$, $R_5CO$ or $NR_6R_7$ groups; |
| Y is | $CF_3$, halogen or phenyl optionally substituted with one to three $C_1$-$C_3$alkyl, $C_1$-$C_3$alkoxy, $C_1$-$C_3$alkylthio, $C_1$-$C_3$alkylsulfinyl, $C_1$-$C_3$alkylsulfonyl, halogen, CN, $NO_2$, $CF_3$, $R_4CF_2B$, $R_5CO$ or $NR_6R_7$ groups; |
| Z is | halogen or $CF_3$; |
| $R_1$ is | hydrogen, $C_1$-$C_6$alkyl or $C_3$-$C_6$cycloalkyl; |
| $R_2$ and $R_3$ are | each independently hydrogen, $C_1$-$C_6$alkyl or $C_3$-$C_6$cycloalkyl |
| $R_4$ is | hydrogen, fluorine, $CHF_2$, CHFCl or $CF_3$; |
| $R_5$ is | $C_1$-$C_3$alkyl, $C_1$-$C_3$alkoxy or $NR_6R_7$; |
| $R_6$ is | hydrogen or $C_1$-$C_3$alkyl; |
| $R_7$ is | hydrogen, $C_1$-$C_3$alkyl or $R_8CO$; |
| $R_8$ is | hydrogen or $C_1$-$C_3$alkyl; |
| B is | $S(O)_q$ or O; |
| m, n, p and q are | each independently an integer of 0, 1 or 2 with the proviso that the sum (p + m) must be greater than 0; |

Q is 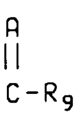, 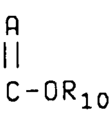, 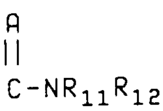, 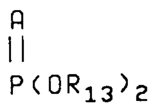,

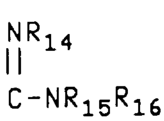, 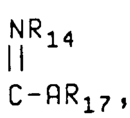, 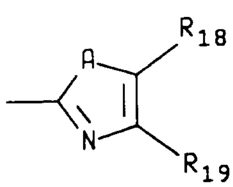, 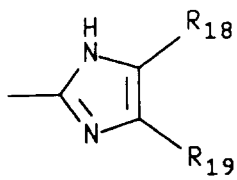,

CN, $C_1$-$C_6$alkyl optionally substituted with one or more phenyl, CN or halogen groups or phenyl optionally substituted with one to three $C_1$-$C_3$alkyl, $C_1$-$C_3$alkoxy, halogen, CN, $NO_2$, $CF_3$ or $NR_{20}R_{21}$ groups;

| | |
|---|---|
| A is | O or S; |
| $R_9$ is | $C_1$-$C_6$alkyl or phenyl; |
| $R_{10}$ is | $C_1$-$C_6$alkyl; |
| $R_{11}$ and $R_{12}$ are | each independently hydrogen, $C_1$-$C_6$alkyl or $-(CH_2)_n-$ wherein n is an integer of 4, 5 or 6 to form a 5- to 7- membered ring; |
| $R_{13}$ is | $C_1$-$C_4$alkyl; |
| $R_{14}$ is | hydrogen, $C_1$-$C_4$alkyl or $-(CH_2)_n$ wherein n is an integer of 4, 5 or 6 when taken together with either $R_{15}$ or $R_{17}$ to form a 5- to 7- membered ring optionally substituted with one or two $C_1$-$C_3$alkyl groups; |
| $R_{15}$ and $R_{16}$ are | each independently hydrogen or $C_1$-$C_4$alkyl; |

$R_{17}$ is $C_1$-$C_4$alkyl or $-(CH_2)_n$ wherein n is an integer of 4, 5 or 6 when taken together with $R_{14}$ to form a 5- to 7- membered ring optionally substituted with one or two $C_1$-$C_3$alkyl groups;

$R_{18}$ and $R_{19}$ are each independently hydrogen or $C_1$-$C_3$alkyl or when taken together may form a ring wherein $R_{18}R_{19}$ is represented by -CH=CH-CH=CH-;

$R_{20}$ and $R_{21}$ are each independently hydrogen or $C_1$-$C_3$alkyl and

the acid addition salts thereof.

**2.** The compound according to claim 1 having the structure

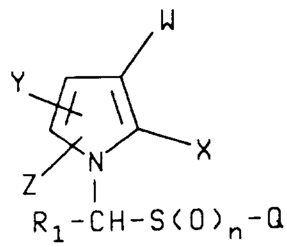

**3.** The compound according to claim 1, [3-bromo-5-(p-chlorophenyl)-4-cyano-2-(trifluoromethyl)pyrrol-1-yl]dimethylthiocarbamic acid, methyl ester.

**4.** A method for the control of insect, acarid or mollusk pests which comprises contacting said pests or their food supply, habitat or breeding grounds with a pesticidally effective amount of a compound having the structure

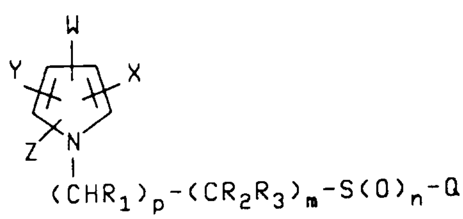

I

wherein W, X, Y, Z, $R_1$, $R_2$, $R_3$, m, n, p, and Q are described in claim 1.

**5.** The method according to claim 4 wherein W is CN; X is halogen or phenyl optionally substituted with one to three halogen, CN or $CF_3$ groups; Y and Z are each independently halogen or $CF_3$ and n is O.

**6.** A method for the prevention, control or amelioration of a disease caused by a phytopathogenic fungus which comprises contacting said fungus with a fungicidally effective amount of the compound according to claim 1.

**7.** A method for the prevention, control or amelioration of a disease caused by a phytopathogenic fungus which comprises contacting said fungus with a fungicidally effective amount of the compound according to claim 2 wherein W is CN; X is halogen or phenyl optionally substituted with one to three halogen or $CF_3$ groups; Y and Z are each independently halogen; $R_1$ is hydrogen; n is O and Q is CN, phenyl optionally substituted with one to three halogen or $CF_3$ groups or

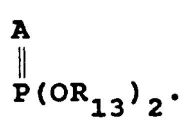

**8.** A method for the protection of growing plants from attack or infestation by insect, acarid or mollusk pests which comprises applying to the foliage of said plants, or to the soil or water in which they are growing, a pesticidally effective amount of a compound having the structure

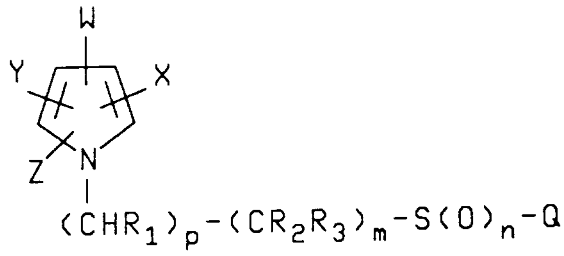

I

wherein W, X, Y, Z, $R_1$, $R_2$, $R_3$, m, n, p and Q are described in claim 1.

**9.** A method for the protection of a plant, plant seed or tuber from fungal infestation and disease which comprises applying to the plant, plant seed or tuber, or to the soil or water in which they are growing, a fungicidally effective amount of the compound according to claim 1.

**10.** A composition for controlling fungal infestation, or insect, acarid or mollusk pests which comprises an inert liquid or solid carrier and a pesticidally effective amount of a compound having the structure

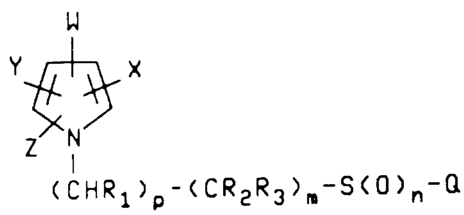

I

wherein W, X, Y, Z, $R_1$, $R_2$, $R_3$, m, n, and p and Q are as described in claim 1.

**11.** A composition for controlling phytopathogenic fungi which comprises an inert liquid or solid diluent and a fungicidally effective amount of a compound having the structure

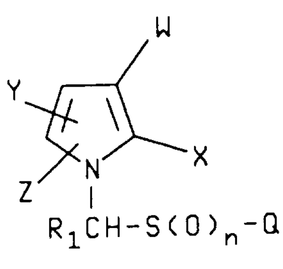

wherein W is CN; X is halogen or phenyl optionally substituted with one to three halogen or $CF_3$ groups; Y and Z are each independently halogen; $R_1$ is hydrogen; n is O and Q is CN, phenyl optionally substituted with one to three halogen or $CF_3$ groups or

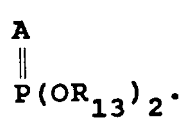

**12.** A molluscicidal bait composition which comprises an edible nutritive substance, a carbohydrate source and a molluscicidally effective amount of a compound having the structure

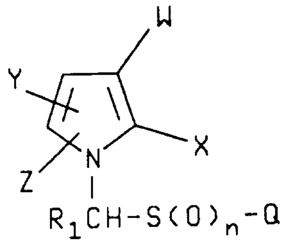

wherein W is CN; X is halogen; Y and Z are each independently halogen or $CF_3$; and $R_1$, n and Q are as described in claim 1.

**13.** A process for the preparation of a compound of formula I

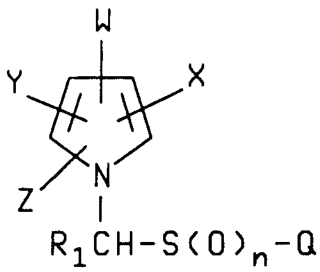

wherein W, X, Y, Z and $R_1$ are as described in claim 1, n is O and Q is

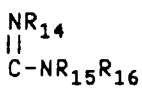, 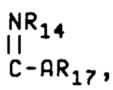, 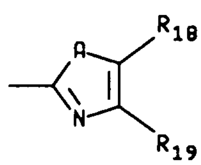 or 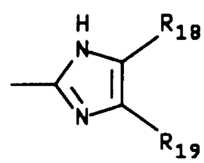

in which A, $R_{14}$, $R_{15}$, $R_{16}$, $R_{17}$, $R_{18}$ and $R_{19}$ are as described in claim 1 which comprises reacting a compound of formula III

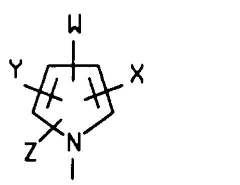

III

with at least one molar equivalent of a thiourea reagent of formula X

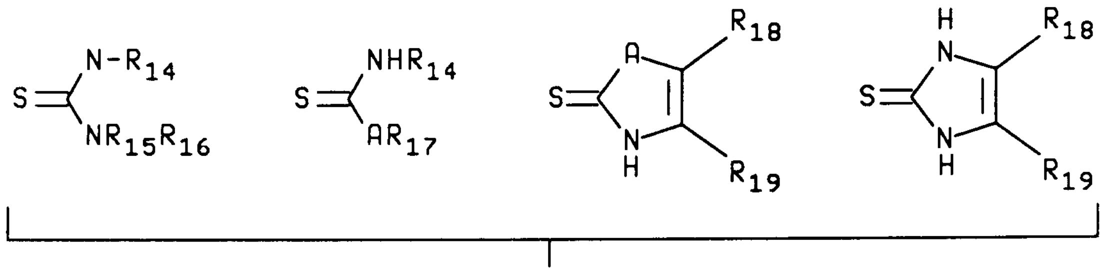

X

in the presence of a solvent.

**14.** A process for the preparation of a compound of formula I

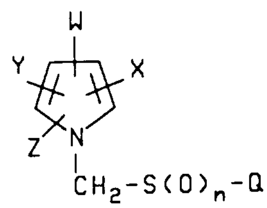

I

wherein W, X, Y and Z are as described in claim 1, n is O and Q is $C_1$-$C_6$alkyl optionally substituted with one or more phenyl, CN or halogen groups or phenyl optionally substituted with one to three $C_1$-$C_3$alkyl, $C_1$-$C_3$alkoxy, halogen, CN, $NO_2$, $CF_3$ or $NR_{20}R_{21}$ groups in which $R_{20}$ and $R_{21}$ are independently hydrogen or $C_1$-$C_3$alkyl which comprises reacting a compound of formula IV

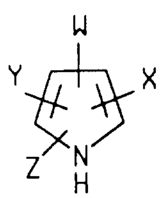

IV

with at least one molar equivalent of a halomethyl thioether reagent of formula XI

$Q$-$S$-$CH_2$-halogen XI

wherein Q is defined hereinabove in the presence of a base and a solvent.

European Patent Office

# EUROPEAN SEARCH REPORT

Application Number

EP 92 11 9141
PAGE1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 312 723 (AMERICAN CYANAMID CO)<br>* page 6, line 20; page 7, line 20; example 9, page 23, table, entries 11, 14; claims 1-4, 6, 8, 9* | 1,2,4,5, 8,10,14 | C07D207/34<br>C07D207/42<br>C07D403/12<br>C07D413/12<br>C07D417/12<br>C07F9/572<br>A01N43/36<br>A01N43/50<br>A01N43/54<br>A01N43/76<br>A01N43/78<br>A01N43/52<br>A01N43/48<br>A01N47/14<br>A01N47/42<br>A01N47/06<br>A01N47/16<br>A01N57/08 |
| X | EP-A-0 347 488 (AMERICAN CYANAMID CO)<br>* page 6, line 56; page 7, line 56; example 9, page 27, table, entries 11, 14; claims 1, 4, 6, 8, 9 * | 1,2,4,5, 8,10,14 | |
| D | & US-A-5 010 098 | | |
| X | EP-A-0 358 047 (AMERICAN CYANAMID CO)<br>* example 9, page 19, table, entries 11, 14; claims * | 1,2,6,9, 14 | |
| A | DE-A-1 814 335 (TENNECO CHEMICALS INC)<br>* examples 4, 10, 19, 21, 34; claims 1-3, 6, 9 * | 1,2,4, 6-11 | **TECHNICAL FIELDS SEARCHED (Int. Cl.5)** |
| A | DE-A-1 695 906 (TENNECO CHEMICALS INC)<br>* examples 4, 23, 30, 36, 41, 43; claims 1-3, 10 * | 1,2,4, 6-11 | C07D<br>C07C<br>C07F<br>A01N |
| A | DE-A-1 816 740 (TENNECO CHEMICALS INC)<br>* examples 4, 13, 14, 16, 21, 23; claims 1, 9 * | 1,2,4, 6-11 | |
| A | EP-A-0 372 263 (AMERICAN CYANAMID INC)<br>* claim 1, in particular page 55, line 12; claims 2, 4, 8, 9, 11-18, 20, 21 * | 1,4,8, 10,12,14 | |

-/--

The present search report has been drawn up for all claims

| Place of search | Date of completion of the search | Examiner |
|---|---|---|
| BERLIN | 17 FEBRUARY 1993 | VAN AMSTERDAM L. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 92 11 9141
PAGE2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 182 737 (CIBA-GEIGY AG)<br>* table 3, compound 3.183; claims 1-3, 8, 15-18 *<br>----- | 1,6,9,10 | |
| | | | TECHNICAL FIELDS SEARCHED (Int. Cl.5) |

The present search report has been drawn up for all claims

| Place of search | Date of completion of the search | Examiner |
|---|---|---|
| BERLIN | 17 FEBRUARY 1993 | VAN AMSTERDAM L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document